# EUROPEAN PATENT APPLICATION

(11) **EP 1 267 299 A2**
(43) Date of publication of application: **18.12.2002**
(21) Application number: 02254122.1
(22) Date of filing: 13.06.2002
(51) Int. Cl.: G06F 19/00

(54) **Online fracture management system and associated method**

(30) Priority: 14.06.2001 US 298243 P; 28.06.2001 US 301640 P; 03.05.2002 US 138012
(71) Applicant: DEPUY ORTHOPAEDICS, INC., Warsaw, Indiana 46581 (US)
(72) Inventor: Turley, Troy, Warsaw, IN 46581 (US); Hershberger, Lesa, Warsaw, IN 46582 (US); Wadsworth, Barry, Warsaw, IN 46582 (US)
(74) Representative: Belcher, Simon James

(57) **Abstract**

An orthopaedic component for use in an orthopaedic procedure is selected in a procedure which involves the steps of determining a user-selected image of an orthopaedic anomaly and generating an image-selected control signal in response thereto. An orthopaedic component for treating the orthopaedic anomaly from a plurality of orthopaedic components is determined in response to the image-selected control signal.

## Description

The present invention relates generally to online systems, and more particularly to an online fracture management system and associated method for use in assisting a surgeon in the selection of an implantable orthopaedic component. The invention provides apparatus, a method, a system, and a program for providing an interactive environment for selection of a medical device such as an implantable orthopaedic component.

In the field of orthopaedics, surgeons are typically required to utilize traditional techniques in regard to the selection of an implantable orthopaedic component. In particular, the surgeon will generally be presented with a catalogue or the like from the various component manufacturers which list the available implantable components for use in a surgical procedure. For example, a catalogue may include a list of the various bone plates and bone screws which are available from a particular manufacturer. Moreover, the catalogue may include a brief description of the features of a particular plate or screw. However, by definition, a printed publication is "static" in nature and therefore does not allow for interaction between the surgeon and the component manufacturer in regard to the selection of the proper component for use in the treatment of a given patient.

Moreover, a number of online lists have also been created by component manufactures. Specifically, a number of manufacturers of orthopaedic components maintain websites or the like which allow a user to peruse through the various products offered by the manufacturer. In particular, heretofore designed websites have typically included a listing of the manufacturer's products organized by a particular product category. For example, a list of each of the manufacturer's available bone screws may be listed on a webpage and available for viewing by a user if the user clicks or otherwise indicates that he or she desires to view a list of the manufacturer's bone screws. Hence, in this manner, the online lists offered on the websites of component manufacturers are quite similar in nature to their previously described printed counterparts. Specifically, such online lists are static in nature in the sense that such lists do not provide for any dynamic interaction in regard to assisting the surgeon in selection of the proper orthopaedic component.

What is needed therefore is a system and method which overcomes one or more of the above-mentioned drawbacks. What is specifically needed is a system and method which provides for dynamic interaction between the surgeon and the component manufacturer in regard to the surgeon's selection of an implantable orthopaedic component for use in the treatment of a given patient.

In one aspect, the invention provides apparatus for selecting an orthopaedic component for use in an orthopaedic procedure, the apparatus comprising:
a processing unit,
a memory electrically coupled to the processing unit, the memory having stored therein a plurality of instructions which, when executed by the processing unit, causes the processing unit to:
   determine a user-selected image of an orthopaedic anomaly and generate an image-selected control signal in response thereto, and
   determine an orthopaedic component for treating the orthopaedic anomaly from a plurality of orthopaedic components in response to the image-selected control signal.

In another aspect, the invention provides a method for selecting an orthopaedic component for use in an orthopaedic procedure, comprising the steps of:
determining a user-selected image of an orthopaedic anomaly and generating an image-selected control signal in response thereto, and
determining an orthopaedic component for treating the orthopaedic anomaly from a plurality of orthopaedic components in response to the image-selected control signal.

The apparatus of the invention can be used to perform the method of the invention.

In a further aspect, the invention provides an article comprising a computer-readable signal-bearing medium, the medium comprising a plurality of instructions which, when executed by a processing unit, causes the processing unit to determine a user-selected image of an orthopaedic anomaly and generate an image-selected control signal in response thereto, and to determine an orthopaedic component for treating the orthopaedic anomaly from a plurality of orthopaedic components in response to the image-selected control signal.

The article of the invention can be used in conjunction with the apparatus of the invention. The article of the invention can be used in performing the method of the invention.

According to one illustrative embodiment, there is provided a dynamic system and method for assisting a surgeon or other user in the selection of an implantable orthopaedic component for use in the treatment of a patient.

In a more specific illustrative embodiment, there is provided a network-based system and method which allow a surgeon to navigate through a number of menus or graphical representations displayed on a display screen or other output device in order to determine the appropriate orthopaedic component for use with a given patient. In one implementation of this embodiment, the surgeon is presented with a number of images on a display screen with each of such images being representative of a different orthopaedic anomaly. For instance, each of the images may depict a varying degree or location of bone fracture of a particular bone which corresponds to the fractured bone that the surgeon is treating. In any event, the surgeon may compare the displayed images to a representation of the patient's condition (e.g., the radiology films of the patient's injury or ailment) and thereafter select the displayed image that most closely resembles the patient's condition. Based on the selection input by the surgeon, a particular type and/or configuration of orthopaedic component may be selected and displayed to the surgeon thereby instructing the surgeon on the proper choice of component.

In a more specific exemplary embodiment, the previously described system and method is made available to the surgeon via a global network such as the internet. Specifically, the surgeon may use a client machine such as a personal computer (PC) to communicate with a server on which the files and databases which provide the previously described functions reside. In this exemplary embodiment, connection to the server from the surgeon's PC is provided via the internet.

In regard to another exemplary embodiment, in addition to providing the surgeon with the identity of an orthopaedic component which may be utilized in the treatment of the patient, additional information may also be provided to the surgeon based on the surgeon's selection. For example, a surgical technique instruction guide may be presented to the surgeon by either displaying the same on the display screen or printing a copy of the same on a printer. Moreover, information regarding the surgical instruments for use to implant the selected orthopaedic component may also be presented to the surgeon.

In yet another exemplary embodiment, a number of additional menus or graphical representations may be utilized to assist the surgeon in the navigation of the displayed information. For example, an initial image of the entire skeletal system may be displayed to the surgeon. The surgeon may then select a particular portion of the skeletal system (e.g., a particular bone) and thereafter select a particular subportion of the skeletal system (e.g., a particular region or structure of a particular bone).

In another exemplary embodiment, it should be appreciated that additional data may be input by the surgeon in order to further enhance the capabilities of the system in identifying the proper implantable component. For example, in addition to selecting the image which most closely resembles the anomaly (e.g., the fracture) presented by the patient, the surgeon may also input additional information about the patient and the patient's skeletal structure such as age, bone porosity condition, and size. These additional inputs would then be factored into the selection of the implantable orthopaedic component offered by the system to the surgeon for use in treating the patient.

Embodiments of the present invention will now be described by way of example with reference to the accompanying drawings, in which:
FIG. 1 is a simplified block diagram of a network-based system which incorporates the features of the present disclosure therein; and
FIGS. 2-8 illustrate various screen displays which are displayed on a display monitor during operation of the system of FIG. 1.

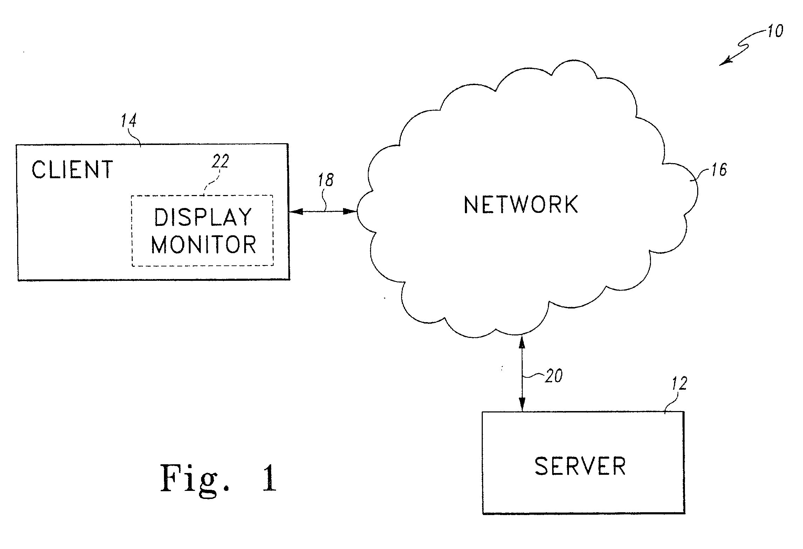

FIG. 1 shows a network-based system 10 having a network server 12 which communicates with a client 14 via a network 16. Although only one network server 12 and one client 14 are shown in FIG. 1, it should be appreciated that the system may include any number of servers 12 or clients 14.

In a conventional manner, each of the servers 12 and the client machines 14 includes a number of components commonly associated with such machines. For example, although not shown in detail in the drawings, each of servers 12 and the clients 14 may include, amongst other things customarily included in such machines, a central processing unit ("CPU"), a non-volatile memory such as a read only memory ("ROM"), a volatile memory such as a random access memory ("RAM"), and one or more data storage devices. It should also be appreciated that such components may be integrated into a single housing or may be provided as a number of separate, discrete devices. It should also be realized that the server 12 and the client 14 may be operated with known, commercially available software operating systems.

As such, the server 12 may be provided as any type of commercially available server. The storage devices associated with the server 12 are provided to maintain a number of databases and files which are utilized in the construction and operation of an information portal such as a website.

The client 14 preferably includes an output device such as a display monitor 22 for displaying a number of images to a user. As such, the client 14 may be embodied as any type of commercially available computing device such as a personal computer ("PC"). Moreover, the client 14 may also be embodied as a "mobile" device such as a cellular phone, a mobile data terminal, a portable computer, a personal digital assistant ("PDA"), or some other device of similar kind.

As shown in FIG. 1, the server 12 is coupled to the network 16 via a communications link 18, whereas the client 14 is coupled to the network 16 via a communications link 20. It should be appreciated that the communications links 18, 20 may be provided as any number of different types of data links including both wired and wireless data links. Moreover, it should also be appreciated that one or more intervening modems (not shown), data routers (not shown), and/or internet service providers ("ISPs") (not shown) may be used to transfer the data between the server 12, the client 14, and the network 16.

The network 16 of the present disclosure may be embodied as any type of network such as a LAN or WAN. Moreover, in a specific illustrative embodiment, the network 16 is embodied as a publicly-accessible global network such as the internet.

A user such as surgeon may utilize the client machine 14 in order to access information stored on the server 12 (or on a device associated with the server 12). In the case of an internet-based system (i.e., the network 16 is embodied as the internet), the server 12 is utilized as a web server and, as such, hosts a website which may be accessed by the surgeon from the client 14. In doing so, a number of files in the form of, for example, webpages may be downloaded from the server 12 to the client 14 via the network 16 for display to the surgeon on the display monitor 22. The surgeon may then peruse the contents of the displayed pages and select certain portions thereof in order to gain information.

In an exemplary embodiment, the methods, apparatuses, systems, and programs described herein may be utilized to assist a user such as a surgeon in the selection of an implantable orthopaedic component for use in the treatment of a patient. In particular, data downloaded from the server 12 to the client 14 may be utilized to display a number of menus or graphical representations in the form of webpages on the display monitor 22 of the client 14. The surgeon may navigate through such menus or graphical representations in order to determine the appropriate orthopaedic component for use with the treatment of the given patient. In one specific implementation of this embodiment, a number of images indicative of different orthopaedic anomalies are displayed on the display monitor 22 for review by the surgeon. For instance, each of the images may depict a varying degree or location of bone fracture of a particular bone which corresponds to the bone fracture the surgeon is treating.

The surgeon may compare each of the images displayed on the display monitor 22 to a representation of the patient's condition (e.g., the radiology films of the patient's injury or ailment) and thereafter select the displayed image that most closely resembles the patient's condition by touching a particular key or "clicking" on the displayed image with an input device such as a mouse. The client 14 generates an output signal indicative of the surgeon's selection and transmits the same to the server 12 via the network 16. It should be appreciated that additional data may be input by the surgeon in order to further refine the selection of the proper implantable component. For example, in addition to selecting the image which most closely resembles the anomaly (e.g., the fracture) presented by the patient, the surgeon may also input additional information about the patient and the patient's skeletal structure such as age, bone porosity condition, and size.

In any event, the server 16 then analyses the inputted data (i.e., the selected image and any additional information) from the surgeon and determines an appropriate response thereto. In particular, the server 12 may search a number of databases or other data repositories in order to determine a particular orthopaedic component that is best suited to treat the particular anomaly identified by the surgeon. Once a particular orthopaedic component (or components) has been selected based on the selection by the surgeon, data files associated with a particular type and/or configuration of orthopaedic component are retrieved from a database and transmitted to the client 14 via the network 16. The client 14 then utilizes the contents of such files to display to the surgeon a number of images (both text and graphical) associated with the selected orthopaedic component.

In addition to providing the surgeon with the identity of an orthopaedic component to utilize in the treatment of the patient, additional information may also be retrieved by the server 12 and transmitted to the client 14 for presentation to the surgeon. For example, a surgical technique instruction guide may be presented to the surgeon by either displaying the text of such a guide on the display monitor 22 or presenting a downloadable file which may be downloaded and subsequently printed by use of a printer (not shown) associated with the client 14. Moreover, information regarding the surgical instruments for use to implant the orthopaedic component may also be transmitted from the server 12 to the client 14 for presentation to the surgeon.

It should be appreciated that a number of pages may be displayed on the display monitor 22 of the client 14 in order to further assist the surgeon in selecting the proper orthopaedic component. For example, an initial image of the entire skeletal system may initially be displayed on the display monitor 22 of the client 14. The surgeon may then select a particular portion of the skeletal system (e.g., a particular bone). A control signal indicative of the surgeon's selection is generated and transmitted to the server 12 via the network 16. The server 12 may then utilize the contents of the transmitted signal to determine the next page to display to the surgeon. For example, if the surgeon selected a particular bone, an image of each of the subportions of such a bone may be displayed to the surgeon for further selection thereof.

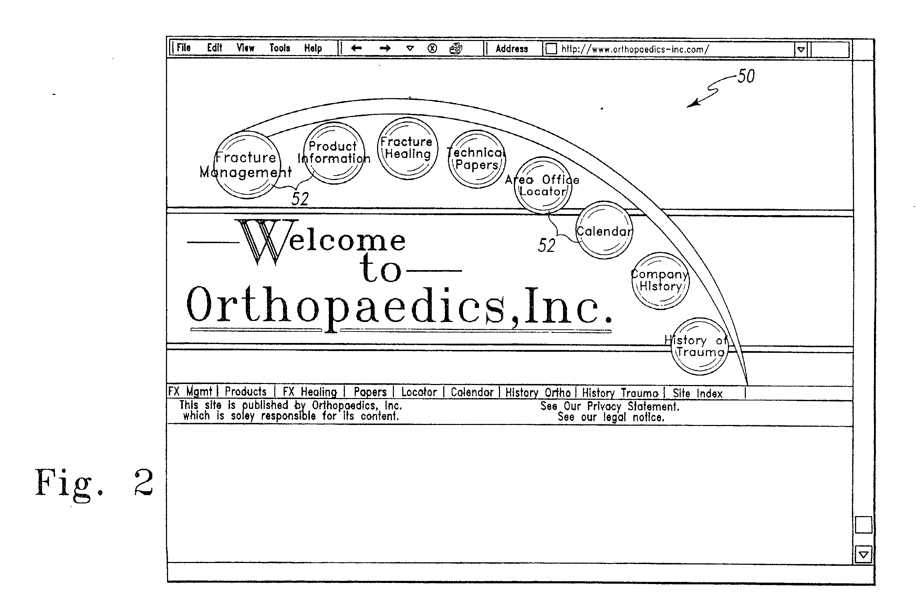

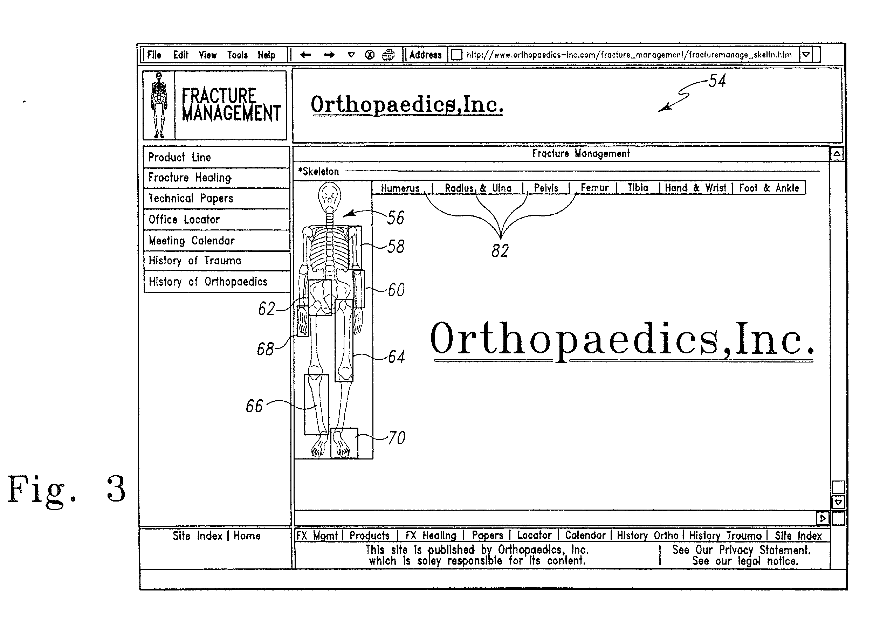

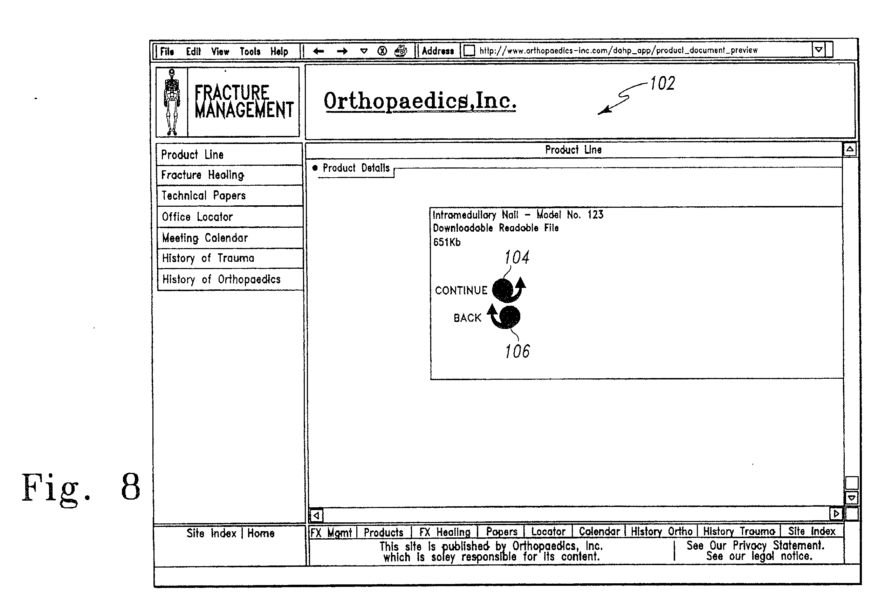

Referring now to FIGS. 2 to 8, there is shown a number of screen displays in the form of webpages which demonstrate the previously described concepts in the context of a specific exemplary embodiment. As shown in FIG. 2, data files are downloaded from the server 14 which cause an initial page 50 to be compiled and displayed on the display monitor 22 of the client 14. The page 50 has a number of initial navigation images in the form of links such as "buttons" 52. The buttons 52 are indicative of the different areas of the website which may be navigated by the surgeon. When the user clicks or otherwise selects a particular navigation button 52, such as the one exemplary identified as "Fracture Management" in FIG. 1, a control signal is generated and transmitted to the server 12 which causes data files associated with a second page 54 to be downloaded from the server 12 to the client 14. The client 14 then utilizes the downloaded data files to generate and display the images associated with the page 54 on the display monitor 22 of the client 14 (see FIG. 3).

The page 54 includes an image of a human skeleton 56 having a number of selectable portions highlighted thereon. In the exemplary embodiment described herein, the skeleton 56 includes a selectable portion indicative of a humerus 58, a radius and ulna 60, a pelvis 62, a femur 64, a tibia 66, a hand and wrist 68, and a foot and ankle 70. It should be appreciated that the particular selectable portions described herein are merely exemplary in nature and that any number of selectable portions may be utilized. Moreover, the selectable portions may include portions of the skeleton 56 other than the individual bones. For example, joint locations such as the hip and shoulder may be identified with selectable portions on the displayed image of the skeleton 56.

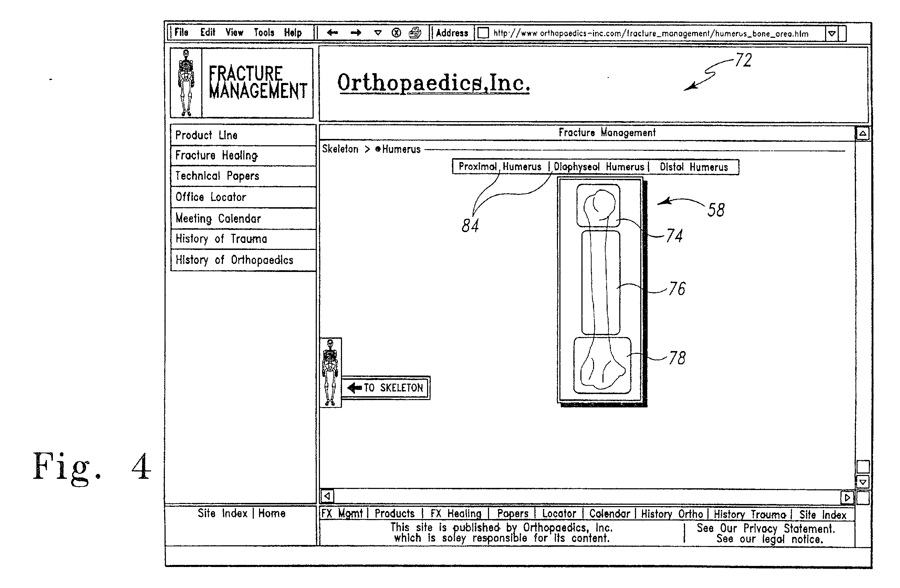

When the user clicks or otherwise selects a particular selectable portion of the skeleton 56, a control signal is generated by the client 14 and transmitted to the server 12 thereby causing data files associated with a third page 72 to be downloaded from the server 12 to the client 14. The client 14 then utilizes the downloaded data files to generate and display the images associated with the page 72 on the display monitor 22. For example, if the surgeon selects the selectable portion associated with the humerus 58, data files associated with the humerus 58 are downloaded to the client 14 thereby causing the client 14 to display the same on the display monitor 22 (see FIG. 4).

It should be appreciated that the user may navigate to the desired subsequent page (e.g., the page 72) by use of locations of the page 54 other than the selectable portions of the skeleton 56. For example, as shown in FIG. 3, the page 54 may also be configured to include a number of individually-labelled buttons 82 which perform substantially the same function as the previously described selectable portions of the skeleton 56. For example, if the user selects the button 82 labelled "Humerus", the page 72 (see FIG. 4) is loaded much in the same way as if the user had clicked on the selectable portion of the skeleton 56 corresponding to the humerus 58.

As shown in FIG. 4, the page 72 includes an image of the selected portion (e.g., the selected bone) having a number of selectable portions highlighted thereon. In the exemplary embodiment described herein, the image displayed is that of the humerus 58 (which was selected form the page 54). The humerus includes a selectable portion indicative of the proximal humerus 74, the diaphyseal humerus 76, and the distal humerus 78. Again, it should be appreciated that the particular selectable portions described herein are merely exemplary in nature and that any number of selectable portions of the selected bone may be utilized.

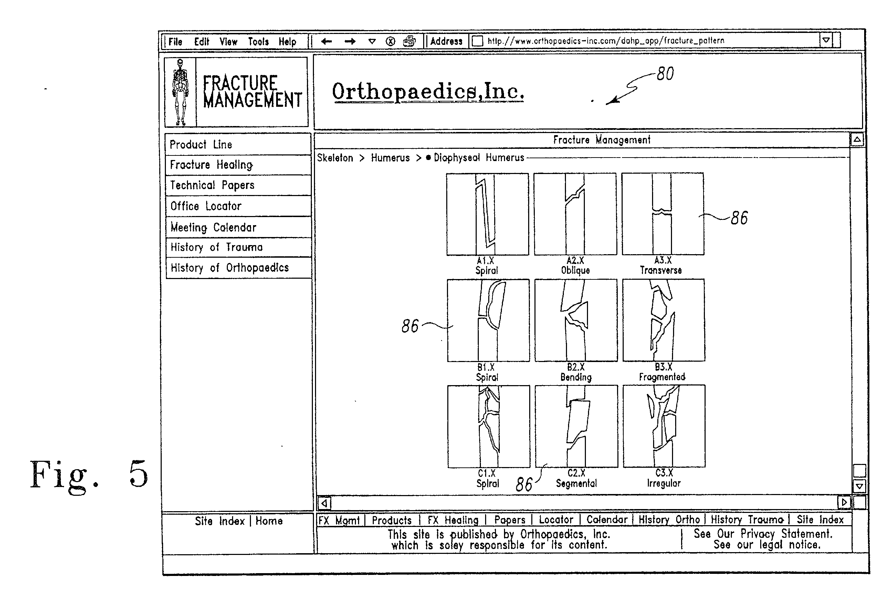

When the user clicks or otherwise selects a particular selectable portion of the humerus 58, a control signal is generated by the client 14 and transmitted to the server 12 thereby causing data files associated with a fourth page 80 to be downloaded from the server 12 to the client 14. The client 14 then utilizes the downloaded data files to generate and display the images associated with the page 80 on the display monitor 22 of the client 14. For example, if the surgeon selects the selectable portion associated with the diaphyseal humerus 76, data files associated with the diaphyseal humerus 76 are downloaded to the client 14 thereby causing the client 14 to display the same on the display monitor 22 (see FIG. 5).

Similarly to as described above, it should be appreciated that the user may navigate to the desired subsequent page (e.g., the page 80) by use of locations of the page 72 other than the selectable portions of the humerus 58. For example, as shown in FIG. 4, the page 72 may also be configured to include a number of individually-labelled buttons 84 which perform substantially the same function as the previously described selectable portions of the humerus 58. For example, if the user selects the button 84 labelled "Diaphyseal Humerus", the page 80 (see FIG. 5) is loaded much in the same way as if the user had clicked on the selectable portion of the humerus 58 corresponding to the diaphyseal humerus 76.

As shown in FIG. 5, the page 80 includes a number of images of the selected portion of the selected bone, each of which is indicative of a different type or location of an orthopaedic anomaly. In the exemplary embodiment described herein, a number of images 86 indicative of different fracture types of the diaphyseal humerus 76 are displayed. For example, images 86 associated with various types of spiral, oblique, transverse, bending, fragmented, segmental, and irregular fractures of the diaphyseal humerus 76 are displayed. It should be appreciated that the particular types of fractures described herein are merely exemplary in nature and that any number of different types of fractures may be displayed.

The surgeon then compares the fracture to the patient that the surgeon is treating with the displayed images 86. In particular, the surgeon may determine which displayed image 86 most closely matches or resembles the patient's fracture being treated by the surgeon. Thereafter, the surgeon clicks or otherwise selects the selected image 86 that most closely matches or resembles the fracture of the surgeon's patient thereby causing a control signal to be generated by the client 14. Such a control signal is transmitted to the server 12.

The server 12 then analyses the content of the received signal from the client 14 in order to determine an appropriate response thereto. In particular, the server 12 may search a number of databases or other data repositories in order to determine a particular orthopaedic component that is best suited to treat the particular anomaly (e.g., fracture) identified in the image 86 that was selected by the surgeon. Once a particular orthopaedic component (or components) has been selected, data files associated therewith are retrieved from a database or other data repository and downloaded from the server 12 to the client 14. The client 14 then utilizes the downloaded data files to generate and display the images associated with a fifth page 88 on the display monitor 22 of the client 14.

For example, if the surgeon selects the image 86 associated with a transverse fracture of the diaphyseal humerus 76 (see FIG. 5), the server 12 searches a number of databases or other data repositories in order to determine a particular orthopaedic component (or number of components) that is best suited to treat a transverse fracture of the type indicated by the surgeon (i.e., of the type depicted in the selected image 86). Once a particular number and type of orthopaedic components have been selected based on the input by the surgeon, the associated data files are retrieved from the database or other data repository. The retrieved files are then downloaded from the server 12 to the client 14. The client 14 then utilizes the downloaded data files to generate and display the images associated with the fifth page 88 on the display monitor 22 of the client 14.

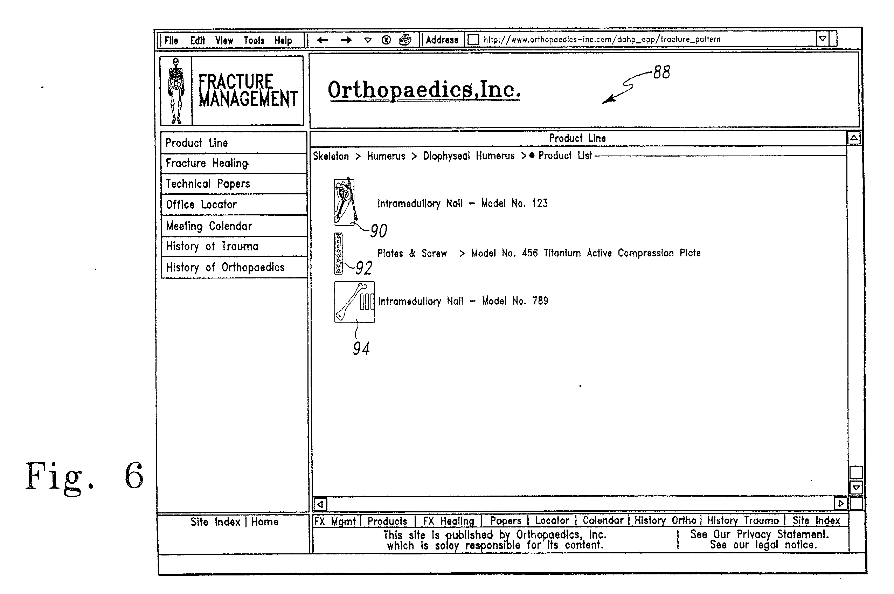

As shown in FIG. 6, the page 88 includes a number of images of the orthopaedic components which may be utilized to treat the particular type of fracture indicated by the surgeon. In the exemplary embodiment described herein, the images displayed on the page 88 may include an image of a first type of intramedullary nailing system 90, an image of a plate and screw system 92, and an image of a second type of intramedullary nailing system 94. Again, it should be appreciated that the particular types of components described herein are merely exemplary in nature and that any number or type of components for treating the identified fracture may be utilized.

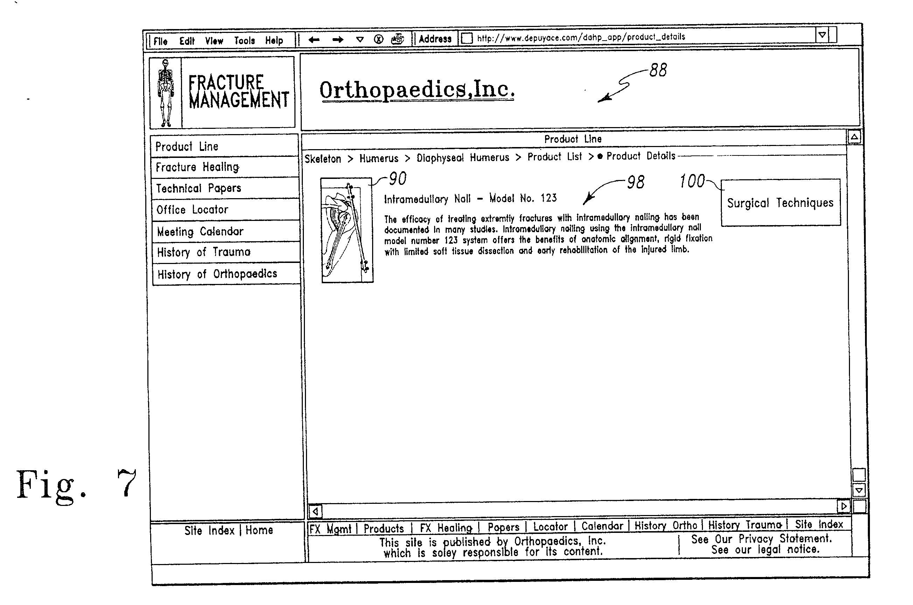

The surgeon may then decide on a particular type of orthopaedic device from the options presented on the page 88 and thereafter procure the same for use in the treatment of the surgeon's patient. It should be appreciated that, as shown in FIG. 7, if a surgeon desires additional information regarding one or more of the suggested components, the surgeon may click on or otherwise select the image of the component thereby causing a page 96 to be downloaded, compiled, and displayed. For example, if the surgeon desires more information regarding the first type of intramedullary nailing system 90, the surgeon may click on the image of the same on page 88 thereby causing the page 96 to be generated and displayed in a manner similar to as described above in regard to other pages. As shown in FIG. 7, the page 96 includes descriptive information 98 relating to the selected component.

As also shown in FIG. 7, the page 96 may be configured to include a link in the form of a button 100. If the surgeon desires a downloadable and printable file containing, for example, the surgical technique instruction guide relating to the selected orthopaedic component, the surgeon may click on the button 100. In doing so, a page 102 is downloaded, compiled, and displayed on the display monitor 22 of the client 14. The page 102 has a link in the form of a button 104 which allows the surgeon to download the printable file. If the surgeon changes his or her mind (presumably after seeing the displayed file size), the surgeon may select a button 106 which allows him or her to return to the page 96.

As alluded to above, it should be appreciated that additional data may be input by the surgeon in order to further refine the selection of the proper implantable component. For example, in addition to selecting the one of the images 86 which most closely resembles the anomaly (e.g., the fracture) presented by the patient, the surgeon may also input additional information about the patient and the patient's skeletal structure such as age, bone porosity condition, and size. Such information may be entered at the beginning of the surgeon's use of the system (e.g., prior to selecting a portion of the skeleton 56 from the page 54). Alternatively, the surgeon may enter such additional information at a point in the process nearer the time in which the surgeon selects one of the images 86. For example, the page 80 may be configured to include a number of menus, links, or text entry areas which may be utilized by the surgeon to enter such additional information.

There are a plurality of advantages of the present disclosure arising from the various features of the apparatus, methods, systems, and programs described herein. It will be noted that alternative embodiments of each of the apparatuses, methods, systems, and programs of the present disclosure may not include all of the features described yet still benefit from at least some of the advantages of such features.

For example, although the software concepts disclosed herein are described as already being loaded or otherwise maintained on a computing device (e.g., either a client or server machine), it should be appreciated that the present disclosure is intended to cover the software concepts described herein irrespective of the manner in which such software concepts are disseminated. For instance, the software concepts of the present disclosure, in practice, could be disseminated via any one or more types of a recordable data storage medium such as a modulated carrier signal, a magnetic data storage medium, an optical data storage medium, a biological data storage medium, an atomic data storage medium, and/or any other suitable storage medium.

## Claims

1. Apparatus for selecting an orthopaedic component for use in an orthopaedic procedure, the apparatus comprising:
a processing unit,
a memory electrically coupled to the processing unit, the memory having stored therein a plurality of instructions which, when executed by the processing unit, causes the processing unit to:
determine a user-selected image of an orthopaedic anomaly and generate an image-selected control signal in response thereto, and
determine an orthopaedic component for treating the orthopaedic anomaly from a plurality of orthopaedic components in response to the image-selected control signal.

2. The apparatus of claim 1, wherein the plurality of instructions, when executed by the processing unit, further causes the processing unit to generate an image of the orthopaedic component for treating the orthopaedic anomaly.

3. The apparatus of claim 1, further comprising a display monitor electrically coupled to the processing unit, wherein the plurality of instructions, when executed by the processing unit, further causes the processing unit to display an image of a human skeleton on the display monitor, and to determine a user-selected portion of the human skeleton.

4. The apparatus of claim 1, further comprising a display monitor electrically coupled to the processing unit, wherein the plurality of instructions, when executed by the processing unit, further causes the processing unit to display an image of a human skeleton on the display monitor, to determine a user-selected bone of the human skeleton and generate a bone-selected control signal in response thereto, and to display a plurality of images indicative of orthopaedic anomalies of the user-selected bone on the display monitor in response to the bone-selected control signal.

5. The apparatus of claim 4, wherein the plurality of instructions, when executed by the processing unit, further causes the processing unit to generate the image-selected control signal in response to a user's selection of the user-selected image of the orthopaedic anomaly from the plurality of images indicative of orthopaedic anomalies.

6. The apparatus of claim 1, further comprising a display monitor electrically coupled to the processing unit, wherein the plurality of instructions, when executed by the processing unit, further causes the processing unit to display an image of a human skeleton on the display monitor, to determine a user-selected bone of the human skeleton and generate a bone-selected control signal in response thereto, and to display an enlarged image of the selected bone showing a plurality selectable anatomic structures of the user-selected bone in response to the bone-selected control signal.

7. The apparatus of claim 6, wherein the plurality of instructions, when executed by the processing unit, further causes the processing unit to determine a user-selected anatomic structure of the user-selected bone and generate a structure-selected control signal in response thereto, and to display a plurality of images indicative of orthopaedic anomalies of the user-selected anatomic structure on the display monitor in response to the structure-selected control signal.

8. The apparatus of claim 7, wherein the plurality of instructions, when executed by the processing unit, further causes the processing unit to generate the image-selected control signal in response to a user's selection of the user-selected image of the orthopaedic anomaly from the plurality of images indicative of orthopaedic anomalies of the user-selected anatomic structure.

9. The apparatus of claim 1, further comprising a display monitor electrically coupled to the processing unit, wherein the plurality of instructions, when executed by the processing unit, further causes the processing unit to retrieve an image data file associated with the orthopaedic component for treating the orthopaedic anomaly from an image database, to generate an image of the orthopaedic component for treating the orthopaedic anomaly based on the image data file, and to display the image of the orthopaedic component for treating the orthopaedic anomaly on the display monitor.

10. The apparatus of claim 9, wherein the image database is maintained on a server machine, and the plurality of instructions, when executed by the processing unit, further causes the processing unit to retrieve the image data file associated with the orthopaedic component from the image database over a network, and to display the image of the orthopaedic component for treating the orthopaedic anomaly on a client machine.

11. The apparatus of claim 10, wherein the plurality of instructions, when executed by the processing unit, further causes the processing unit to retrieve the image data file associated with the orthopaedic component for treating the orthopaedic anomaly over the internet.

12. The apparatus of claim 1, further comprising a display monitor electrically coupled to the processing unit, wherein the plurality of instructions, when executed by the processing unit, further causes the processing unit to display a graphical link to a surgical technique instruction guide associated with the orthopaedic component on the display monitor in response to the image-selected control signal.

13. A method for selecting an orthopaedic component for use in an orthopaedic procedure, comprising the steps of:
determining a user-selected image of an orthopaedic anomaly and generating an image-selected control signal in response thereto, and
determining an orthopaedic component for treating the orthopaedic anomaly from a plurality of orthopaedic components in response to the image-selected control signal.

14. A method as claimed in claim 13, which makes use of apparatus as claimed in any one of claims 1 to 12.

15. An article comprising a computer-readable signal-bearing medium, the medium comprising a plurality of instructions which, when executed by a processing unit, causes the processing unit to determine a user-selected image of an orthopaedic anomaly and generate an image-selected control signal in response thereto, and to determine an orthopaedic component for treating the orthopaedic anomaly from a plurality of orthopaedic components in response to the image-selected control signal.

16. The article of claim 15, which is used in conjunction with apparatus as claimed in any one of claims 1 to 12.

17. The article of claim 15, which is used in a method as claimed in claim 13 or claim 14.
